# EUROPÄISCHE PATENTSCHRIFT

(11) **EP 0 470 108 B1**
(45) Veröffentlichungstag und Bekanntmachung des Hinweises auf die Patenterteilung: **27.09.1995**
(21) Anmeldenummer: 90906194.7
(22) Anmeldetag: 19.04.1990
(51) Int. Cl.: A61L 2/12

(54) **VEREINFACHTES VERFAHREN ZUM STERILISIEREN VON HALBFESTEN BIS FESTEN STOFFZUBEREITUNGEN AUF BASIS VON OLIGOMEREN NIEDERER HYDROXYCARBONSÄUREN ODER DEREN SALZE**
A SIMPLIFIED PROCESS FOR STERILISING SEMI-SOLID TO SOLID PREPARATIONS BASED ON OLIGOMERS OF LOWER HYDROXYCARBOXYLIC ACIDS OR THEIR SALTS
PROCEDE SIMPLIFIE DE STERILISATION DE COMPOSITIONS SEMI-SOLIDES OU SOLIDES A BASE D'OLIGOMERES D'ACIDES HYDROXYCARBOXYLIQUES INFERIEURS OU DE LEUR SELS

(30) Priorität: 28.04.1989 DE 3914080
(43) Veröffentlichungstag der Anmeldung: 12.02.1992
(73) Patentinhaber: Henkel Kommanditgesellschaft auf Aktien, 40191 Düsseldorf (DE)
(72) Erfinder: RITTER, Wolfgang, D-5657 Haan (DE); FUES, Johann-Friedrich, D-4048 Grevenbroich 5 (DE)
(74) Vertreter: von Kreisler, Alek, Dipl.-Chem.
(86) Internationale Anmeldenummer: EP9000627
(87) Internationale Veröffentlichungsnummer: WO9013319

(56) Entgegenhaltungen:
- DE-A- 3 810 284

## Beschreibung

Gegenstand der deutschen Offenlegungsschriften DE 32 29 540 und 37 16 302 sind resorbierbare Wachse zur mechanischen Blutstillung an körpereigenem Hartgewebe, insbesondere an Knochen, die sich dadurch auszeichnen, daß sie aus bei Körpertemperatur zähviskosen bis festen wachsartigen Polyester-Oligomeren von niederen Hydroxycarbonsäuren bestehen. Als besonders geeignet werden entsprechende Polyester, Oligomere der Milchsaure und/oder der Glycolsäure beschrieben. Aufgrund ihrer Struktur sind diese Wachse durch körpereigene Stoffwechselmechanismen abbaubar, wobei die Geschwindigkeit des Abbaus in an sich bekannter Weise einstellbar ist. Die bevorzugten Wachse weisen mittlere Molekulargewichte im Bereich von etwa 200 bis 1500 und insbesondere im Bereich von etwa 300 bis 1000 auf.

Zur Regelung des mittleren Molekulargewichts werden monofunktionelle und/oder difunktionelle Alkohole und/oder Carbonsäuren mitverwendet, wobei dem Glycerin als polyfunktionellem Alkohol besondere Bedeutung zukommen kann. Die resorbierbaren Materialien sind weiterhin in einer wichtigen Ausführungsform von freien Carboxylgruppen praktisch vollständig befreit. Möglich ist das entweder durch eine gezielte Reinigung wie sie beispielsweise in den zuvor genannten Druckschriften beschrieben ist oder aber durch Salzbildung an Carboxylgruppen, die im Reaktionsrohprodukt noch vorliegen oder über beispielsweise freie Hydroxycarbonsäuren der genannten Art gezielt zugesetzt sind.

So beschreiben die älteren deutschen Patentanmeldungen P 38 25 211.2 (D 8293) und P 38 26 915.5 (D 8265) Massen auf Basis von Oligomeren der geschilderten Art, die sich durch einen Gehalt an körperverträglichen Salzen organischer und/oder anorganischer Säuren auszeichnen, wobei entsprechende Salze durch Abreaktion der im Oligomerwachs gegebenenfalls vorliegenden freien Carboxylgruppen ausgebildet und/oder als zugesetzte Salze homogen verteilt in das Wachs eingearbeitet sind. In der zuletzt genannten älteren Anmeldung wird dieses Konzept der körperverträglichen und körperresorbierbaren Oligomerverbindungen insbesondere auf Basis der Glycolsäure und/oder Milchsäure dahingehend erweitert, daß hier wenigstens anteilsweise resorbierbare Knochenersatz- und/oder Knochenverbundwerkstoffe sowie Hilfsstoffe für die Einbindung von Prothesenmaterial in lebendes Knochengewebe beschrieben werden. Die in diesem Zusammenhang eingesetzten Oligomertypen können mittlere Molekulargewichte des erweiterten Bereichs von etwa 200 bis 10000 g/Mol und insbesondere des Bereichs von etwa 300 bis 5000 g/Mol enthalten. Als geeignet sind insbesondere Abmischungen solcher Oligomerharze beschrieben, die keramische Werkstoffe in bevorzugt homogener Verteilung dispergiert enthalten. Diese insbesondere in Pulver- und/oder in Granulatform vorliegenden Keramikwerkstoffe können körperresorbierbar oder auch nicht resorbierbar sein, wobei bioaktiven Keramikwerkstoffen vor allem auf Basis von Calciumphosphatverbindungen besondere Bedeutung zukommen kann. Geeignete Calciumphosphate sind beispielsweise Hydroxylappatit und/oder Tricalciumphosphat.

Die Herstellung der geschilderten Oligomerverbindungen erfolgt durch eine Polykondensations- und/oder Polyadditionsreaktion - in der Regel unter Einwirkung geringer Mengen schwach saurer Katalysatoren - bei erhöhten Temperaturen. Auch die Einarbeitung der gegebenenfalls mitverwendeten keramischen Werkstoffe und/oder Salze erfolgt üblicherweise bei erhöhten Temperaturen, im allgemeinen über die Schmelzphase der organischen Harze.

Für den praktischen Einsatz dieser Massen in der Operationstechnik ist im allgemeinen die portionierte Abpackung bei gleichzeitiger Sicherstellung eines vollsterilen Arbeitsmaterials Voraussetzung. Die Erfindung geht von der Aufgabe aus, diese Voraussetzungen in möglichst einfacher Weise für Materialien der beschriebenen Art zu erfüllen. Die technische Lösung der Erfindung baut dabei auf dem bekannten Prinzip der Keimabtötung durch Erhitzen des zu sterilisierenden Materials auf hinreichend hohe Temperaturen auf. Wesentlich ist für die Lehre der Erfindung die Auswahl des technischen Hilfsmittels, mit dem die Temperaturführung während wenigstens wesentlicher Abschnitte der Materialsterilisierung vorgenommen und kontrolliert wird. Die neue Lehre geht von der Erkenntnis aus, daß es möglich ist, das zu sterilisierende Gut durch Einwirkung von hochfrequenten elektromagnetischen Schwingungen des Mikrowellenbereichs - im allgemeinen Sprachgebrauch als "Mikrowellen" bezeichnet - in vorbestimmbarer Weise auf die erforderlichen Temperaturen zu erhitzen.

Gegenstand der Erfindung ist dementsprechend ein Verfahren zum Sterilisieren von halbfesten bis festen Stoffzubereitungen auf Basis von Oligomeren niederer Hydroxycarbonsäuren oder deren Salze und ihren Umsetzungsprodukten mit Molekulargewichtsregelnden Komponenten, insbesondere 1 - und/oder mehrwertigen Alkoholen bzw. entsprechenden Carbonsäuren, die auch in Abmischung mit körperverträglichen Mineralstoffen vorliegen können, durch Erhitzen auf erhöhte Temperaturen, wobei das Verfahren dadurch gekennzeichnet ist, daß das zu sterilisierende Gut der Einwirkung von Mikrowellen unterworfen wird.

Die Anwendung hochfrequenter elektromagnetischer Schwingungen des oberen Megahertz-Bereiches bis in den mittleren Gigahertz-Bereich ist in den letzten Jahren zunehmend in die Praxis umgesetzt worden. Das für den Haushaltsbereich bekannte Beispiel ist der Mikrowellenherd, der zum raschen durchgreifenden Aufheizen und gegebenenfalls vorherigen Auftauen von vorgefertigten Speisen eingesetzt wird. Die ausgewählte Mikrowellenstrahlung des unteren bis mittleren Gigahertz-Bereiches - beispielsweise des Bereichs von etwa 0,1 bis 300 GHz und bevorzugt des Bereichs von etwa 0,1 bis 30 GHz - regt die im aufzuwärmenden Gut vorliegenden Wassermoleküle an und bewirkt damit die Aufheizung des wasserhaltigen Gutes von innen heraus. Die Durchgriffsmöglichkeit der Strahlung in das Innere des aufzuwärmenden Gutes ist bei niedrigeren Frequenzen des angegebenen Bereiches größer als bei den höheren Frequenzen, vergleiche hierzu beispielsweise "Mikrowellen", Günter Nemitz, München 1980, Seite 155.

Der Einsatz von Mikrowellen ist auch auf verwandten Gebieten vorgeschlagen worden, beispielsweise zum Erhitzen feuchter Textilien, insbesondere auch im Rahmen von Wasch- und Trocknungsprozessen. Stets handelt es sich dabei aber um den Eingriff in wäßrig-feuchtes Gut.

Oligomerharze der von der vorliegenden Erfindung betroffenen Art sind aufgrund ihrer Herstellung als wasserfreie Materialien anzusehen. Bekannt sind dabei Polykondensationstypen dieser Art nicht nur des von der Erfindung betroffenen vergleichsweise beschränkten mittleren Molekulargewichtsbereichs, sondern auch feste Polymere dieser Art mit sehr viel höherem Molekulargewicht. Der Versuch, solche Feststoffmaterialien hohen Molekulargewichts - beispielsweise körperresorbierbares Fadenmaterial auf Basis von Hochpolymeren der Glycolsäure und/oder der Milchsäure - durch Einwirkung von Mikrowellen zu erwärmen oder gar auf Sterilisationstemperatur zu erhitzen, führt zu keinem Ergebnis. Hier wird offensichtlich keine hinreichende Resonanz zwischen an sich polaren Molekülbestandteilen und Energieeinstrahlung ausgelöst.

Überraschenderweise sieht die Situation völlig anders aus, wenn Harze der in den eingangs genannten vorveröffentlichten und älteren Schutzrechten bzw. Schutzrechtsanmeldungen beschriebenen Art Einwirkung von Mikrowellen ausgesetzt werden. Die Einstrahlung von Energie im heute haushaltsüblichen Mikrowellenherd führt zu vergleichsweise rascher Aufheizung der Harze, wobei der für die Sterilisation wesentliche Temperaturbereich von wenigstens etwa 100 ^{o}C um so schneller erreicht wird, je schneller die mit dieser Aufheizung verbundene Erweichung und/oder Verflüssigung der Oligomeren eintritt. Die absatzweise Einstrahlung jeweils gleicher Energiebeträge in ein zunächst wachsartig festes und dann bei steigenden Temperaturen zunehmend weicher bis flüssig werdendes Material zeigt darüber hinaus, daß die Interaktion zwischen dem zu erhitzenden Gut und der eingestrahlten Energie um so wirkungsvoller wird, je beweglicher die Harzbeschaffenheit durch Temperatursteigerung wird. Die mit einer vorbestimmten Energie- und Zeiteinheit einstellbaren Temperatursprünge an der Materialprobe werden beim stufenweisen Übergang von dem festen Material zum geschmolzenen Gut mit zunehmender Weichheit der Materialprobe größer.

Eine Erklärung für die Anwendbarkeit des hier betroffenen Arbeitsmittels Mikrowelle zum thermischen Sterilisieren der erfindungsgemäß betroffenen Harzklasse könnte darin liegen, daß auch bei zunächst wachsartig fest erscheinenden Materialproben hinreichende Anteile an vergleichsweise kurzkettigen Oligomeren vorliegen, die zunächst in eine Wechselwirkung mit der eingestrahlten Energie treten können, sich dabei erhitzen, die unmittelbare Umgebung dabei erweichen und damit zur Erweiterung des ansprechbaren Harzvolumens führen. Entscheidend dürfte auch hier sein, daß der Angriff der Mikrowelle nicht nur an den Außenbereichen des zu erwärmenden Gutes, sondern durchdringend im Gesamtmaterial stattfindet. Sichergestellt ist damit gewissermaßen die Erhitzung des Gutes von innen heraus in seiner Gesamtheit. Es ist einleuchtend, daß damit gerade für die erfindungsgemäße Zielsetzung der Keimabtötung Wesentliches erreicht wird. Das gilt nicht nur für Materialien, die als solche durch die Oligomeren beispielsweise der Glycolsäure und/oder der Milchsäure gebildet werden, sondern gerade auch für die in den genannten älteren Anmeldungen geschilderten Mischprodukte, die keramische Werkstoffanteile aufweisen. Bei inniger Vermengung von Harz und keramischem Werkstoff, der vorzugsweise als feinverteilter Bestandteil vorliegt, gelingt durch das erfindungsgemäße Einwirken der Mikrowelle die durchdringende Erhitzung und damit keimabtötende Sterilisierung auch vergleichsweise dicker Materialschichten. Ersichtlich liegt hier eine wesentliche Vereinfachung für die Bereitstellung praxisgerechter portionierter Packungen in vollsterilisierter Form.

Erfindungsgemäß wird es damit möglich, beispielsweise Knochenwachse oder als Klebehilfe bestimmte Materialien für den medizinischen Einsatz in portionsweiser Aufteilung insbesondere in Form von wenigstens weitgehend geschlossenen Portionspackungen der Keimabtötung zu unterwerfen. Das zu sterilisierende Gut wird dabei in einer Umhüllung aufgenommen, die der Einwirkung von Mikrowellen widersteht und den Durchtritt zuläßt. Bekannt ist aus der Alltagspraxis, daß hierfür insbesondere Kunststoffe, z. B. Polyolefine geeignet sind, die heute bereits in großem Umfang z. B. für die Portionierung von Fertiggerichten zum Aufheizen im Mikrowellenherd Verwendung finden.

Die dem Verfahren zu unterwerfenden Oligomermaterialien sollen vorzugsweise im Temperaturbereich der keimabtötenden Behandlung wenigstens zähviskos fließfähig sein. Knochenwachse und Klebstoffe der geschilderten Art und insbesondere der angegebenen mittleren Molekulargewichtsbereiche sind bei Temperaturen oberhalb von 100 ^{o}C z. B. in, Temperaturbereich bis 130 ^{o}C üblicherweise zum fließfähigen Zustand erschmolzen.

In einer besonderen Ausführungsform der Erfindung kann es zweckmäßig sein, bei Raumtemperatur feste Stoffzubereitungen, die zunächst auf die Einwirkung von energiereicher Strahlung der hier betroffenen Art nur wenig ansprechen, dadurch zu aktivieren, daß Materialien dieser Art in konventioneller Weise beschränkt vorgewärmt werden. Es kann dabei zweckmäßig sein, ein solches Vorwärmen von wenigstens Anteilen der jeweils zu sterilisierenden Materialproben bis in den Bereich einer beginnenden Erweichung vorzunehmen und derart vorbehandeltes Material dann der Mikrowelleneinstrahlung auszusetzen. Eine solche kombinierte Temperaturbehandlung wird allerdings nur in Sonderfällen notwendig.

Der Mikrowelleneinsatz im erfindungsgemäßen Verfahren kann intermittierend oder kontinuierlich sein und dabei eine Verfahrensstufe als Ganzes überstreichen oder auch nur Anteile einer solchen Verfahrensstufe betreffen. Die Steuerung der einzustellenden Temperatur gelingt durch Wahl der Intensität und Zeitdauer der Energieeinstrahlung. Durch intermittierende Einstrahlung mit vergleichsweise geringen Leistungen - beispielsweise bis maximal 200 Watt - erlaubt die Einstellung mäßiger Temperaturen und/oder die Einhaltung eines vorgegebenen Temperaturbereichs über einen längeren Zeitraum. In dem Verfahrensabschnitt der Aufheizung kann es zweckmäßig sein, mit vergleichsweise höherer Leistung - beispielsweise mit bis zu 1000 Watt - kontinuierlich oder absatzweise zu arbeiten.

Die Verweildauer des zu sterilisierenden Gutes im vorbestimmten Temperaturbereich der Keimabtötung richtet sich nach dem vorbekannten Wissen zu Sterilisationsvorgängen der hier geforderten Art. So gilt beispielsweise: Erhitzen auf Temperaturen von wenigstens 100 ^{o}C, vorzugsweise von wenigstens 110 ^{o}C für den zur sicheren Abtötung unerwünschter Verkeimung benötigten Zeitraum. Geeignet ist beispielsweise der Temperaturbereich oberhalb 120 ^{o}C bis 180 ^{o}C für einige Minuten oder auch länger.

Zur Durchführung des erfindungsgemäßen Verfahrens ist es häufig hinreichend, eine vorbestimmte Energiemenge auf die zu sterilisierende und vorzugsweise in der wenigstens weitgehend geschlossenen Portionspackung befindliche Materialmenge abzustrahlen und daraufhin die entsprechend hoch aufgeheizte Materialprobe unter Regulierung bzw. Verzögerung der Abkühlung sich selbst zu überlassen. Ein gegebenenfalls erforderliches völliges Verschließen der jeweiligen Portionspackung kann ohne nochmaligen Materialkontakt erfolgen.

### Beispiele

Als Mikrowellengerät wird ein handelsüblicher Mikrowellenherd der Marke "Siemens" verwendet, der einen zeitgesteuerten Leistungseintrag in den folgenden Stufen vorsieht: 90 W, 180 W, 360 W sowie 600 W. Die abgestrahlte Energie hat eine Frequenz von 2,45 GHz.

Die in den nachfolgenden Beispielen im einzelnen charakterisierten Materialproben liegen jeweils in einer Menge von etwa 30 g in nichtverschlossenen Glasfläschchen vor, die zur Versuchsdurchführung nacheinander jeweils in das Zentrum der Heizzone engesetzt werden.

Die Ausgangstemperatur aller Materialproben beträgt - soweit nicht anderes ausdrücklich angegeben ist - 21 ^{o}C. Mikrowellenenergie wird einstufig oder stufenweise in der in den Beispielen angegebenen Art (gewählter Energieeintrag und Dauer der jeweiligen Heizperiode) eingestrahlt. Im unmittelbaren Anschluß an eine Heizperiode wird die Temperatur im oberen Bereich der jeweiligen Materialprobe durch Eintauchen bzw. Eindrücken eines Labor-Quecksilberthermometers bestimmt. Die nachfolgende(n) Heizstufe(n) werden jeweils im unmittelbaren Anschluß an die vorangegangene Temperaturbestimmung vorgenommen.

Die chemische Zusammensetzung der untersuchten Oligomeren und ihre physikalische Beschaffenheit vor sowie nach dem Energieeintrag sind in den nachfolgenden Beispielen zusammengefaßt.

### Beispiel 1

Bei Raumtemperatur viskos fließfähiges Umsetzungsprodukt auf Basis Glycerin/Glycolsaure/Ölsäure im Molverhältnis 1/3/3.

| Heizstufe | Energieeintrag (W) | Heizdauer (sec.) | Temperatur des bestrahlten Guts ^{o}C |
|---|---|---|---|
| 1. | 600 | 30 | 59 |
| 2. | 600 | 15 | 75 |

Die Materialprobe wird auf eine Anfangstemperatur von 30 ^{o}C gekühlt und einem erneuten zweistufigen Heizzyklus unterworfen. Die ermittelten Werte sind die folgenden.

| Heizstufe | Energieeintrag (W) | Heizdauer (sec.) | Temperatur des bestrahlten Guts ^{o}C |
|---|---|---|---|
| 1. | 600 | 60 | 78 |
| 2. | 600 | 60 | 131 |

### Beispiel 2

### Umsetzungsprodukt auf Basis Glycerin/Glycolsäure/Milchsäure/Ölsäure im Molverhältnis 1/6/6/3

Das Ausgangsmaterial hat bei Raumtemperatur dickviskose Beschaffenheit.

| Heizstufe | Energieeintrag (W) | Heizdauer (sec.) | Temperatur des bestrahlten Guts ^{o}C |
|---|---|---|---|
| 1. | 600 | 45 | 74 |

Das erhitzte Material ist dünnflüssig. Es wird auf eine Einsatztemperatur von 32 ^{o}C gekühlt und dann erneut einer zweistufigen Erwärmung mit dem folgenden Ergebnis ausgesetzt.

| Heizstufe | Energieeintrag (W) | Heizdauer (sec.) | Temperatur des bestrahlten Guts ^{o}C |
|---|---|---|---|
| 1. | 600 | 60 | 76 |
| 2. | 600 | 45 | 113 |

### Beispiel 3

Ein Kondensationsprodukt auf Basis Glycerin/Milchsäure im Molverhältnis 1/4, das bei Einsatztemperatur als zähviskoses Produkt vorliegt, wird einstufig bei einem Leistungseintrag von 600 W für 60 sec. bestrahlt. Die erreichte Endtemperatur liegt bei 158 ^{o}C. Die Materialprobe ist dünnflüssig.

### Beispiel 4

Ein zähviskoses Einsatzmaterial auf Basis Glycerin/Milchsäure im Molverhältnis 1/12 wird einer zweistufigen Erwärmung ausgesetzt. Die Arbeitsbedingungen und -Ergebnisse sind die folgenden.

| Leistungseintrag (W) | Zeitdauer (sec.) | Temperatur ^{o}C | Harzbeschaffenheit |
|---|---|---|---|
| 180 | 60 | 38 | zähviskos |
| 600 | 60 | 138 | dünnflüssig |

### Beispiel 5

Ein wachsartig festes Kondensationsprodukt auf Basis Talgalkohol/Milchsäure im Molverhältnis 1/17 wird jeweils bei einem Leistungseintrag von 600 W in 3 Stufen erwärmt. Dauer der ersten Stufen 50 sec., Dauer der zweiten und dritten Stufe jeweils 60 sec..

Nach Abschluß der ersten Einstrahlungsperiode ist das Probenmaterial so fest, daß eine Temperaturmessung nicht möglich ist. Nach der zweiten Erwärmungsstufe ist eine so weitgehende Erweichung eingestellt, daß das Thermometer in die Wachsmasse eingedrückt werden kann. Zu diesem Zeitpunkt bestimmte Temperatur 55 ^{o}C. Zum Abschluß der dritten Heizstufe ist das Material in einem inhomogenen partiell erschmolzenen Zustand. Die Temperatur in den erschmolzenen Bereichen liegt bei 105 ^{o}C.

### Beispiel 6

Ein wachsartig festes Kondensationsprodukt auf Basis Glycerin/Glycolsäure im Molverhältnis 1/9 wird zweistufig bei einem Leistungseintrag von jeweils 600 W jeweils für 30 sec. bestrahlt.

Ergebnis der ersten Behandlungsstufe: Die Materialprobe ist noch überwiegend fest, jedoch herdweise breiig geschmolzen. In diesen Schmelzbereichen wird eine Temperatur von 74 ^{o}C bestimmt. Nach Abschluß der zweiten Heizperiode ist das Material fast vollständig erschmolzen. Die in der Schmelze bestimmte Temperatur liegt bei 133 ^{o}C.

### Beispiel 7

Ein zähviskos/festes Kondensationsprodukt auf Basis Glycerin/Glycolsäure/Milchsäure im Molverhältnis 1/5/1 wird einstufig bei einem Leistungseintrag von 600 W für den Zeitraum von 45 sec. bestrahlt. Es fällt eine dünnflüssige Schmelze mit einer Temperatur von 126 ^{o}C an.

### Beispiel 8

Ein zähviskos-festes Material auf Basis Glycerin/Glycolsäure/Milchsäure im Molverhältnis 1/2/12 wird zweistufig jeweils für den Zeitraum von 60 sec, bei einem Leistungseintrag von 360 W in der ersten Verfahrensstufe und 600 W in der zweiten Verfahrensstufe behandelt.

Nach der ersten Heizstufe ist die Materialprobe zähviskos und auf ca. 38 ^{o}C aufgewärmt. Das in der nachfolgenden Heizstufe anfallende dünnflüssig erschmolzene Material zeigt eine Temperatur von 162 ^{o}C.

### Beispiel 9

Ein Glyerin/Glycolsäureester im Molverhältnis 1/3 - Einsatzkonsistenz zähviskos - wird einstufig bei einem Leistungseintrag von 360 W für den Zeitraum von 70 sec. bestrahlt. Es fällt eine dünnflüssige Schmelze mit einer Temperatur von 131 ^{o}C an.

### Beispiel 10

Ein oligomeres Kondensationsprodukt auf Basis Glycerin/Glycolsäure/Milchsäure im Molverhältnis 1/6/6 wird einem sechsstufigen Aufheizzyklus bei einem Leistungseintrag von jeweils 600 W in der folgenden Zeitsequenz unterworfen:
1. Stufe: 45 sec., Harzmasse unverändert fest und handwarm
2. Stufe: 30 sec., zähviskose Beschaffenheit, 39 ^{o}C
3. Stufe: 20 sec., Harzmasse partiell erschmelzend, in der Schmelzzone 84 ^{o}C
4. Stufe: 10 sec., weiteres Fortschreiten des Schmelzvorganges, in der Schmelzzone 91 ^{o}C
5. Stufe: 10 sec., Gesamtmasse zähfließend erschmolzen bei 94 ^{o}C
6. Stufe: 20 sec., dünnflüssig tropfende Schmelze bei 119 ^{o}C

## Patentansprüche

1. Verfahren zum Sterilisieren von halbfesten bis festen Stoffzubereitungen auf Basis von Oligomeren niederer Hydroxycarbonsäuren oder deren Salze und ihren Umsetzungsprodukten mit Molekulargewichts-regelnden Komponenten, insbesondere 1- und/oder mehrwertigen Alkoholen bzw. entsprechenden Carbonsäuren, die auch in Abmischung mit körperverträglichen Mineralstoffen vorliegen können, durch keimabtötendes Erhitzen auf erhöhte Temperaturen, dadurch gekennzeichnet, daß das zu sterilisierende Gut der Einwirkung von hochfrequenten Schwingungen des Mikrowellenbereichs (Mikrowellen) unterworfen wird.

2. Verfahren nach Anspruch 1, dadurch gekennzeichnet, daß bei Körpertemperatur zähviskose bis wachsartige Massen insbesondere auf Basis von Oligomeren der Glycolsäure und/oder der Milchsäure bzw. deren Derivaten mit 1- und/oder mehrwertigen Alkoholen oder entsprechenden Carbonsäuren, deren körperverträgliche Salze und/oder deren Abmischungen mit körperverträglichen Mineralstoffen dem Verfahren unterworfen werden.

3. Verfahren nach Ansprüchen 1 und 2, dadurch gekennzeichnet, daß für den medizinischen Einsatz z. B. als Knochenwachs oder Klebehilfe bestimmte Materialien der angegebenen Art in portionsweiser Aufteilung, insbesondere in Form von wenigstens weitgehend geschlossenen Portionspackungen der Keimabtötung unterworfen werden.

4. Verfahren nach Ansprüchen 1 bis 3, dadurch gekennzeichnet, daß Oligomermaterialien dem Verfahren unterworfen werden, die im Temperaturbereich der keimabtötenden Behandlung wenigstens zähviskos fließfähig sind.

5. Verfahren nach Ansprüchen 1 bis 4, dadurch gekennzeichnet, daß bei Raumtemperatur feste Stoffzubereitungen im vorgewärmten Zustand, insbesondere wenigstens im Bereich beginnender Erweichung der Mikrowelleneinstrahlung ausgesetzt werden.

6. Verfahren nach Ansprüchen 1 bis 5, dadurch gekennzeichnet, daß das zu sterilisierende Material einer zeitlich begrenzten kontinuierlichen, vorzugsweise aber einer intermittierenden Einwirkung der Mikrowellen unterworfen wird.

## Claims

1. A process for the sterilization of semisolid to solid preparations based on oligomers of lower hydroxycarboxylic acids or salts or reaction products thereof with molecular weight regulators, more particularly monofunctional and/or polyfunctional alcohols or corresponding carboxylic acids, which may even be present in admixture with body-compatible minerals, by germ-destroying heating to elevated temperatures, characterized in that the material to be sterilized is exposed to the effect of high-frequency oscillations in the microwave range (microwaves).

2. A process as claimed in claim 1, characterized in that compositions viscous to wax-like at body temperature, more particularly based on oligomers of glycolic acid and/or lactic acid or derivatives thereof with monofunctional and/or polyfunctional alcohols or corresponding carboxylic acids, body-compatible salts thereof and/or mixtures thereof with body-compatible minerals, are subjected to the process.

3. A process as claimed in claims 1 and 2, characterized in that materials of the type mentioned intended for medical applications, for example as a bone wax or adhesion aid, are sterilized in portion-controlled form, more particularly in the form of at least substantially sealed portion packs.

4. A process as claimed in claims 1 to 3, characterized in that oligomer materials which are at least viscous and free-flowing in the sterilization temperature range are subjected to the process.

5. A process as claimed in claims 1 to 4, characterized in that preparations solid at room temperature are exposed to the microwave radiation in preheated form and, more particularly, in at least incipiently softened form.

6. A process as claimed in claims 1 to 5, characterized in that the material to be sterilized is subjected to the continuous, but preferably intermittent effect of microwaves for a limited time.

## Revendications

1. Procédé de stérilisation de préparations de substances semi-solides à solides à base d'oligomères d'acides hydroxycarboxyliques inférieurs ou de leurs sels et de leurs produits de réaction avec des composants régulant le poids moléculaire notamment des mono et ou polyalcools ou les acides carboxyliques correspondants, qui peuvent aussi être en mélange avec des substances minérales compatibles avec le corps, par chauffage destructeur des germes à température accrue, caractérisé en ce qu'on soumet le produit à stériliser à l'action des ondes à haute fréquence du domaine des micro-ondes (micro-ondes).

2. Procédé selon la revendication 1, caractérisé en ce qu'on soumet au procédé des masses très visqueuses à cireuses à la température du corps, notamment à base d'oligomères d'acide glycolique et/ou d'acide lactique ou de leurs dérivés avec des alcools mono et/ou polyvalents ou des acides carboxyliques correspondants, leurs sels compatibles avec le corps et/ou leurs mélanges avec des matières minérales compatibles avec le corps.

3. Procédé selon les revendications 1 et 2, caractérisé en ce que pour l'utilisation médicale, on soumet des matériaux définis de la nature indiquée par exemple comme cire d'os ou additif de collage en répartition par portion, notamment au moins sous forme de paquets dosés clos, à la destruction des germes.

4. Procédé selon les revendications 1 à 3, caractérisé en ce qu'on soumet les matériaux d'oligomère au procédé, qui dans le domaine de température du traitement de destruction des germes sont au moins des liquides très visqueux.

5. Procédé selon les revendications 1 à 4, caractérisé en ce qu'on expose des préparations de substance solides à température ambiante à l'état préchauffé, notamment au moins dans le domaine de début de ramollissement au rayonnement des micro-ondes.

6. Procédé selon les revendications 1 à 5, caractérisé en ce qu'on soumet le matériau à stériliser à une action continue limitée dans le temps, mais de préférence à une action intermittente des micro-ondes.
